# EUROPEAN PATENT SPECIFICATION

(11) **EP 0 416 248 B1**
(45) Date of publication and mention of the grant of the patent: **06.12.1995**
(21) Application number: 90113433.8
(22) Date of filing: 13.07.1990
(51) Int. Cl.: A61K 31/44, A61K 31/52

(54) **Use of vitamine B6 for reducing elevated serotonin levels**
Verwendung von Vitamin B6 zur Verminderung von hohem Serotoninspiegel
Utilisation de la vitamine B6 pour réduire le taux élevé de sérotonine

(30) Priority: 11.08.1989 GB 8918368
(43) Date of publication of application: 13.03.1991
(73) Proprietor: VESTA MEDICINES (PROPRIETARY) LIMITED, Micor, Johannesburg 2092 (ZA)
(72) Inventor: Serfontein, Willlem Jacob, 0043 Pretoria (ZA)
(74) Representative: VOSSIUS & PARTNER

(56) References cited:
- WO-A-88/06036
- FR-A- 5 552
- AMERICAN JOURNAL OF CLINICAL NUTRITION, vol. 41, no. 4, April 1985, US; R.D. REYNOLDS et al., pp. 684-688
- INTERNATIONAL JOURNAL FOR VITAMIN & NUTRITION RESEARCH, vol. 58, no. 1, 1988; R. DELPORT et al., pp. 67-72
- JOURNAL OF LABORATORY & CLINICAL MEDICINE, vol. 113, no. 1, January 1989; J.B. UBBINK et al., pp. 15-22
- ANNALS OF ALLERGY, vol. 65, no. 1, July 1990; M.R. WEIR et al., pp. 59-62
- INTERNATIONAL JOURNAL FOR VITAMIN & NUTRITION RESEARCH, vol. 60, no. 1, 1990; R. DELPORT et al., pp. 35-40
- ENZYME, vol. 43, no. 2, 1990, CH; J.B. UBBINK et al., pp. 72-79
- DATABASE WPIL, Derwent Publications Ltd., London (GB); AN 91-361699 (49)

## Description

The present invention relates to a new use of vitamin B6 as an active ingredient in a pharmaceutical composition, more particularly where such Vitamin B6 is used in combination with xanthine-type bronchodilators in compositions and methods for the treatment and prophylaxis of obstructive air passage diseases, in particular bronchial asthma.

In WO-A-88 06036 pharmaceutical compositions for treating obstructive air passage diseases are disclosed comprising in combination a xanthine, preferably theophylline in its non-ethylene diaminated form and vitamin B6. Specific embodiments include riboflavin and zinc ion. The ingredients are preferably formulated in a slow release form. The vitamin B6 may be in the form of pyridoxine (PN). An embodiment for treating acute cases is adapted for infusion and contains 50 to 100% of the vitamin B6 in the form of pyridoxal (PL). Ascorbic acid is included as an antioxidant. The zinc ion can be complexed with vitamin B6. Daily dosage rates for human adults are 5 to 500 mg PN and 50 to 600 mg theophylline.

The present invention improves and adds to the teaching of WO-A-88 06036 subject to whatever modifications are proposed in the present specification.

Apart from their bronchodilatory effect xanthine bronchodilators are pharmacologically quite different from other known bronchodilators. They even produce antiasthmatic effects different from purely bronchodilatory effects.

Disadvantages of xanthine bronchodilators include typical CNS side effects, namely:- depression, adverse effects on mood and memory, anxiety and tremors, and high doses may precipitate life threatening seizures.

Another disadvantage of xanthine bronchodilators has been their widely observed diminishing clinical efficacy after prolonged administration of these drugs. This necessitated a progressive increase in dosage rates, which in turn increases the aforesaid side effects. The reason for this phenomenon had not been known. The present invention throws new light on this.

Moreover, adverse side effects of xanthine bronchodilators e.g. aminophylline and theophylline are severely potentiated by many drugs which may be given to asthma patients in combination with xanthine bronchodilatory therapy, e.g. other bronchodilators, gentamycin (in case of infection), dopamine (in case of shock); digoxin; this potentiating effect may result in acute toxic effects and even death. These adverse phenomena must be considered in so serious a light that the advisability of conventional theophylline therapy becomes questionable in the circumstances referred to and in view of the new findings on which this application is based, unless special countermeasures are taken which form the subject of the present application.

In recent years a new potentially useful xanthine bronchodilator has been investigated, known as enprofylline (3-propylxanthine). It differs from theophylline in that none of the nitrogen atoms of the xanthine skeleton are substituted except the N3 position which carries a propyl group (instead of the methyl group of theophylline). Enprofylline is reported to have a bronchodilatory and anti-asthmatic effect, several times stronger than that of theophylline and not to suffer from some of the aforesaid side effects or not to the same degree from many of the aforesaid side effects (Carl G. A. Persson, Asthma Reviews Vol 1 (1987) ISBN 0-12-040921-6, p61-94). However, in all of this work no cognisance was taken of the interaction of xanthine bronchodilators with B6 metabolism as herein further disclosed. The applicant's research, contrary to what one might expect from the aforegoing, has revealed that side effect inducing mechanisms common to xanthine bronchodilators in general also apply to enprofylline, wherefore the present invention is applicable also to therapeutic compositions or preparations and methods involving the use of enprofylline.

In our aforesaid WO-A-8806036 patent application we refer to some prior art involving combinations of theophylline and derivatives thereof and vitamin B6, for the relief of respiratory distress, cardial pulmonary insufficiency and general cardiac complaints.

The aforegoing prior art combinations of xanthine bronchodilators with vitamin B6 were both proposed at a time when little was known about the mechanism of vitamin B6 activity, and even less about its interaction with drugs and chemical substances in the body or of the extremely important role of the pyridoxine activating enzymes in the body (a kinase and an oxidase enzyme) and the effects of disease processes and drugs on these enzymes.

None of the above prior art in any way deals with the reduction or elimination of side effects of xanthine bronchodilators or the enhancement of their efficacy in obstructive air passage therapy, in particular the management of bronchial asthma.

Moreover, this prior art does not teach the correct dosage levels of therapeutic substances required for those purposes.

It is an object of the invention to intervene beneficially in certain newly discovered mechanisms which are responsible for or contribute to the reduced efficacy of xanthine bronchodilators after prolonged use and for various side effects of xanthine bronchodilators.

It is a further object of the present invention to counteract the side effects of xanthine bronchodilators, e.g. of theophylline and of enprofylline in asthma and other obstructive air passage therapy. It is a further or alternative object to potentiate the beneficial effects of xanthine bronchodilators and in particular theophylline and enprofylline in such therapy to permit better therapeutic effects to be attained with a given xanthine bronchodilator dosage, alternatively to permit lowering the therapeutic dosage of such xanthine bronchodilators and lowering the side effects thereof.

The present invention provides a new use of vitamin B6 as an active ingredient in a pharmaceutical composition characterised in that said composition contains an effective amount of said vitamin B6 formulated to counteract elevated serotonin levels in plasma and urine and serotonin associated side effects arising in the treatment or prophylaxis of obstructive air passage disease with a xanthine bronchodilator, such as
a) decreased responsiveness to the xanthine bronchodilator;
b) CNS side effects.

The invention is based on the most surprising discovery that the prolonged administration of xanthine bronchodilators results in substantially increased serotonin levels in human plasma and urine. Serotonin is a powerful bronchoconstrictor. It therefore directly opposes the desired bronchodilatory effect of the xanthine bronchodilator. This explains the observed declining efficacy after prolonged administration of the drug. It is also found surprisingly that these increased serotonin levels are reversed immediately after commencing administration of vitamin B6 at the levels taught by the present invention in order to manage plasma pyridoxal and intracellular phosphorylated pyridoxal.

This observation is rendered particularly surprising in the light of the earlier described CNS effects. The xanthine-induced side effects observed in the CNS are normally consistent with lowered serotonin levels in the brain (which is separated from the plasma and remainder of the body by the blood/brain barrier). It thus appears that the reversal of CNS related side effects by vitamin B6 is due to an interesting interaction of mechanisms.

Yet a further unexpected mechanism has been found to be involved in the benefits derived according to the invention. The prolonged administration of xanthine bronchodilators has now been found to also result in some patients in increased histamine levels, although this effect was not such a constant feature of xanthine administration as are the raised Sertonin levels arising from such treatment. Histamine is a well known inducer of allergic reactions, notably asthma. These increased histamine levels are also reversed in certain cases by vitamin B6 supplementation. Accordingly the new use according to the present invention also serves to prevent or counteract elevated histamine levels induced by the xanthine bronchodilator when they occur.

The observation regarding raised levels of serotonin and histamine and the reversals thereof by vitamin B6 supplementation were all the more unpredictable since serotonin is formed from tryptophan and histamin is formed from histidine by decarboxylation reactions both of which require the presence of vitamin B6.

Yet a further new use according to the invention is that it also serves to prevent or counteract elevated homocysteine levels and effects thereof, which sometimes occur, after administration of xanthine bronchodilators. This feature of the invention is based on one of the most recent and quite surprising research findings, namely that xanthine bronchodilators in some patients raise plasma homocysteine levels and that this as well is normalised by the invention. Homocysteine in plasma is highly toxic, and elevated levels thereof will not only contribute to some of the known clinical effects of xanthine bronchodilators but will cause additional harmful effects in the long term which had not been previously reported in the context of xanthine bronchodilators. These side effects include deteriorations of various tissue, including CNS, connective tissue, skeletal tissue and particularly vascular tissue. Homocysteine also has a prothrombic effect.

In addition, the invention may be used to prevent or counteract xanthine bronchodilator-induced lowering of glutamate pyruvate transaminase in red blood cells.

The inventor carried out clinical tests in human volunteers to investigate the effect of theophylline on glutamate pyruvate transaminase (GPT) in red blood cells, which plays an important biochemical role. The GPT content of cells was found to be dependent on the amount of PLP present at the time that the enzyme was formed, i.e. during the process of RBC regeneration in the bone marrow. When theophylline was administered there took place a dramatic drop in both PLP due to the inactivation of the kinase, and after approximately 28 days, also of RBC GPT levels as newly formed RBC (produced in the bone marrow under conditions of reduced PLP availability) begin to appear in the circulation.

As predicted, the body did indeed try to compensate for this by forming progressively more kinase. In spite of this the PLP content and the GPT content remained depressed for as long as 100 days after commencing theophylline treatment. Thus the increased levels of kinase were unable to reverse the theophylline induced PLP deficiency and the concommittant GPT deficiency. This demonstrates a need for the intracellular PLP shortage to be compensated for in an appropriate manner from the beginning of therapy with theophylline (or equivalent xanthine bronchodilators).

Surprisingly this can be achieved (as taught by the present invention) with relatively low levels of supplementation with Vitamin B6 preferably never exceeding 200 mg/day, preferably much less, and generally well below the dosages of the xanthine bronchodilator, the effect being much superior, if the Vitamin B6 is administered in a slow-release (sustained release) form.

Surprisingly and most importantly, this low dosage supplementation with Vitamin B6 not only almost immediately reversed the PLP deficiency induced by the xanthine bronchodilator but at the same time reversed the side effects of the xanthine bronchodilator on the central nervous system (CNS), such as restlessness, seizures, depression, headaches, nausea, tremor, especially in children.

The invention also teaches that preferably the vitamin B6, preferably in a slow or sustained release form, is in the form of pyridoxal, preferably in combination with an antioxidant, or a compound which in vivo, once it has entered the bloodstream, is rapidly converted into pyridoxal without the intervention of oxidase or oxygen, preferably pyridoxal itself or a complex or acid addition thereof, or is in the form of pyridoxine or a complex or acid addition salt of pyridoxine, provided that such pyridoxine, or complex or addition salt thereof is always galenically presented in a slow or sustained release form.

In accordance with preferred embodiments of the invention the vitamin B6 is provided in a combination with the xanthine bronchodilator, as dosage units, but as separate and distinct compounds, and the dose by weight of vitamin B6 is substantially lower than that of the xanthine bronchodilator, preferably in a ratio of vitamin B6 to xanthine bronchodilator of 1:2 to 1:70, more preferably 1:8 to 1:30, and wherein preferably the vitamin B6 as well as the xanthine bronchodilator are incorporated in a slow or sustained release form.

A most important observation is that all the stated benefits of vitamin B6 supplementation require relatively low dosage rates of vitamin B6, usually only a small fraction of the dosage rates of xanthine bronchodilator, but made available to the body at as even a rate as possible, e.g. by slow sustained release. For reasons explained further below, excess dosages of vitamin B6, particularly when in the form of pyridoxine can be harmful in that they can produce adverse effects similar to those of a vitamin B6 shortage.

As far as can be established, the aforesaid phenomenon are common to the entire class of xanthine bronchodilators to a greater or lesser extent. The applicant's investigations have shown that enprofylline, which was believed to be almost free from adverse side effects, has an adverse effect on vitamin B6 levels in human plasma and cells. More particularly enprofylline has been found to interfere with the enzymes responsible for the maintenance of adequate vitamin B6 activities in the body. This was not to be expected. This observation confirms that enprofylline therapy as well can benefit from the teachings of the present invention.

Both substances, the xanthine bronchodilator, e.g. theophylline and vitamin B6 are used within the previously accepted conventional dosage range limits. The dosage rate for the xanthine, in particular theophylline can optionally be lowered even, due to the beneficial effect of vitamin B6 supplementation.
However, in accordance with the present invention and in the context set out in the opening paragraph, it has now been found that for prolonged treatment, particularly if pyridoxine (which is less preferred than pyridoxal) is to be the source of vitamin B6, the optimal dosage rates for vitamin B6 are much lower than was previously suspected. Whilst the minimum daily intake for pyridoxine, known as "recommended daily allowance" (RDA) is approx. 2 mg for average adults, a maximum prolonged daily intake of 50 mg should not be exceeded, and particularly not in bolus form. In slow release form about double to triple that amount can be tolerated by most adults without apparent adverse effects, but should nevertheless be avoided in prolonged use. However, such high dosages have now been recognised to be generally wasted, being in excess of the enzymatic capacity of the liver to convert the pyridoxine (PN) into pyridoxal (PL), the substance which by way of the plasma has to enter the various body cells where it is then to be converted by phospho-kinase into phosphorylated pyridoxal (PLP), the actual active form of vitamin B6 inside cells. If the excess of pyridoxine is not excreted sufficiently rapidly, pyridoxine will accumulate in the plasma (where its concentration is normally extremely low or zero) albeit for short periods only and will then diffuse into the cells of the body together with pyridoxal made available by the plasma and become metabolically trapped there. Inside the cell the pyridoxine will then compete against pyridoxal for the limited amount of available kinase and will thus competitively inhibit the conversion of pyridoxal into phosphorylated pyridoxal, the only form in which vitamin B6 is active and useful inside the cells. This effect is aggravated by the fact that the xanthine bronchodilators, e.g. theophylline, which have to be administered as well in accordance with the present invention, as a side effect to their desirable therapeutic effects, have the property of inhibiting phospho-kinase inside the cells where PLP has to be formed. An overdose of pyridoxine is accordingly undesirable for achieving the desired combination effect aimed at by the present invention. This stresses the importance of administering the pyridoxine in accordance with the invention at a rate which does not exceed materially the enzymatic saturation level of the liver for pyridoxine and preferably in slow release form to avoid transient high levels of pyridoxine especially inside peripheral cells.

For the above stated reasons the present invention teaches that the vitamin B6, particularly if it is pyridoxine but even when in the form of pyridoxal should be administered, preferably in slow release or sustained release form, at an (adult) daily rate of 5 mg to 50 mg, preferably 10 mg to 40 mg, optimally 20 mg to 30 mg, say 25 mg. These rates may be increased, e.g. doubled at the beginning of treatment (loading dose).

More specifically the vitamin B6, particularly if present as the less preferred pyridoxine (PN), is in a slow- or sustained-release form. Preferably the xanthine bronchodilator, preferably theophylline or enprofylline, is also present in a slow-release or sustained release form. For example, the product comprises a slow-release form of PN and a slow or sustained-release form of theophylline in a single oral capsule,tablet or tabule. The following relevant experimental facts, now surprisingly found by us, confirm and stress the advantage of supplementing vitamin B6 in slow-release form combined with the xanthine bronchodilator in the same preparation or separately, preferably also in a slow-release form:-
a) The fact that theophylline and other xanthines including enprofylline specifically supress the enzyme PL-kinase. Sustained low levels of theophylline will have less of an enzyme suppressing effect than massive bolus doses especially since the type of inhibition is of the non-competitive variety.
b) The modest affinity that the enzyme PL-kinase has for its substrate PL. This implies that lowered intracellular PL levels such as may occur in disease, including asthma, will adversely affect production of intracellular PLP. Maintenance of moderately elevated intracellular PL levels over longer periods is clearly advantageous.
c) We have demonstrated that very low doses of PN (0,02 mg/kg) in humans have a profound influence on B6 dependent intracellular pathways without affecting extracellular PL and PLP levels materially.

The slow release forms are preferably formulated to release more than 90% of the vitamin B6, e.g. pyridoxine in between 4 and 10 hours, more preferably 6 to 10 hours, say about 8 hours.

It will now become apparent why a dosage ratio of 1:1 as recommended in some prior art preparations is quite unsuitable for the purposes of the present invention, because the correct therapeutic dosage rates for the xanthine bronchodilators to be employed in accordance with the invention are several times higher than those for pyridoxine. Taking theophylline, a preferred xanthine bronchodilator according to the invention, as a basis, the continuous daily dosage rates are (for adults of 70 kg) in the range of 200 to 1400 mg, preferably 400 to 1200 mg, more preferably 600 to 1000 mg, and typically 650 to 800 mg per day, spaced preferably as evenly as possible over the day, and again administered preferably in sustained release or slow release form substantially as defined in the context of pyridoxine. In this context it is pointed out that the release rate of pyridoxine need not necessarily be proportional to that of the xanthine type drug. Indeed, in practice, in certain preferred embodiments the pyridoxine release is completed before the theophylline release has been completed.

The daily dosage rate of xanthine bronchodilator (e.g. of theophylline) per kg will thus be (on the above basis):
2,7 to 20,0 mg/kg/day, preferably 7,1 to 17,1 mg/kg/day, more preferably 8,6 to 14,3 mg/kg/day, and typically 9,3 to 11,4 mg/kg/day.

However, it is stressed that the blood levels of patients receiving a given daily dosage of theophylline will vary in practice quite considerably from one individual to another. The present invention teaches that ideally a blood level of 10 to 20 mg/l should be maintained. Preferably this blood level should be monitored (as is readily done in modern pathological laboratories) and the daily dosage should be adjusted accordingly. This is facilitated by using singly or preferably multiply scored tablets or tabules.

However, as a general guide line the majority of adults (60 to 70 kg) taking 375 mg of theophylline twice daily in slow release form have been found to have theophylline blood levels in the desired range of 10 to 20 mg/l.

It is preferred to build up the slow release daily dosage rate of xanthine bronchodilator over a period, starting with dosage rates of about 1/3 to ½ the final dosage rate and monitoring blood levels after each dose adjustment.

In the compositions the bronchodilator content is at least twice and up to two orders of magnitude as high as, but preferably not more than 200 times the content of Vitamin B6.

It will be understood that the choice of ratio depends inter alia on the bronchodilatory and antiasthmatic activity of the selected xanthine bronchodilator.

In addition, for maximum effect, either or preferably both of two further optional ingredients should preferably be included, namely zinc (e.g. as zinc chloride) and riboflavin, the latter particularly if pyridoxine is used as source of vitamin B6. Both act as potentiators for pyridoxine activation and are particularly effective when present in combination. Zinc ions act as a co-factor in the PL-kinase which is responsible for the phosphorylation of PL to PLP. Supplementation of these substances is highly recommended, because these substances are often not present in sufficient quantities in the body of the patient for vitamin B6 to become fully effective. In many patients the activity levels of the oxidase enzyme (required for the oxidation of pyridoxine to pyridoxal) are sub-optimal due to a deficiency of riboflavin.

In addition to theophylline, enprofylline is another preferred xanthine bronchodilator which, contrary to prior art opinion, shares properties with other xanthine bronchodilators in the context of which Vitamin B6 supplementation is beneficial, involving mechanisms or combinations of mechanisms inapplicable to the prior art or not previously taken into account correctly or at all:
a) Surprisingly, xanthine bronchodilators in general, including theophylline and enprofylline, and not only aminophylline (but by a mechanism not previously known and different from that known for aminophylline or piperazine derivatives) cause in humans substantial and clinically important lowering of B6 blood and cellular levels.
b) The mechanism by which xanthine bronchodilators containing no ethylene diamino moiety, e.g. theophylline depress plasma B6 in humans is not due to Schiff base formation and aldamine complexes or equivalent reactions but rather to the inhibition of enzymes, namely the enzyme PL-kinase, associated with B6 activation leading to low intra-cellular PLP levels. This was not previously known or taken into consideration in the formulation of xanthine-containing drug formulations.
c) This has direct practical clinical consequences: it means that to overcome this enzyme block, the substrate PL concentration must be increased intracellularly and not extracellularly where chemical deactivation could take place.
d) Some of the important side effects previously ascribed to theophylline toxicity are in fact not due to theophylline primarily but rather to concommitant depressed intracellular B6 levels.
e) Low B6 levels in asthmatic patients not on xanthine bronchodilator therapy are due to excessive production of biogenic amines and/or other disease produced compounds which inactivate either kinase activity or oxidase activity or both.
f) Various different and partly interconnected mechanisms exist by means of which B6 assists the asthmatic patient namely :
   (i) depression of xanthine-induced levels of serotonin, which is a broncho constrictor, thereby improving the long-term efficacy of xanthine bronchodilators.
   (ii) counteracting xanthine-induced histamine formation if and when this occurs
   (iii) reversing xanthine-induced CNS side effects.
   (iv) altered oxygen delivery to tissues
   (v) increased respiratory muscle contractions (increased diaphragm contractility)
   (vi) minimisation of supposedly xanthine-associated side effects.
   (vii) effects on catecholamine synthesis (adrenaline)
g) The abovementioned CNS side effects in patients on xanthine bronchodilators are mainly due to low intracellular active B6 (PLP) levels. This finding was not to be expected in the light of incorrect prior art findings that theophylline does not lower plasma PLP and that on the contrary plasma PLP is sporadically increased. The latter effect, we now realise, is due to PLP being released from its albumin complexes by xanthine bronchodilators. However, different known mechanisms prevent this PLP from directly becoming available inside the tissue cells.
h) This also explains why the side effects, e.g. of theophylline are potentiated by certain drugs such as gentamycin and dopamine, which are known B6 antagonists, and by conditions leading to excessive production of biogenic amines (physiological shock, cell death in severely ill patients).
i) Even theophylline as such - by mechanisms different from those described above for its ethylene diamino or piperazine derivatives - antagonises vitamin B6, namely by enzyme inhibition, in particular a kinase inhibition, and this has to be taken into account in clinical situations since the consequences of this effect are mainly manifested intracellularly and are of special significance in the long term administration of theophylline such as in the treatment of bronchial asthma and other obstructive air passage diseases. This is especially so since the inactivation of the enzyme is of the non-competitive type.
j) PL kinase inhibition depresses not only the intracellular PLP levels, but also the ratio of PLP level to PL level in the cells, since PN is converted into PL in the liver almost entirely where, as also in the serum and in other tissues, it is reversibly phosphorylated to PLP with the aid of PL kinase. Since PL but not PLP is capable of crossing other cell membranes, it is important for maximal effect of a vitamin B6 supplement to boost the PL substrate level inside the cells to, and maintain it at, moderately elevated levels. This is attained maximally by supplementing the vitamin B6 at a slow and uniform rate, particularly when the source of vitamin B6 is PN, since PN is rapidly cleared from the plasma (t_{½}= 12 min), while the decay of plasma PL levels, although somewhat slower, also occurs relatively rapidly (t_{½} = 30 - 40 min). Daily bolus doses of PN would therefore result in large plasma PL fluctuations.
k) Also, the uptake of PL from the plasma into cells and intracellular conversion into PLP is a saturable process at relatively low external (i.e. in the plasma) PL concentrations. Hence it is important to raise the PL level in the plasma and to maintain such moderately elevated levels over many hours.
l) The avoidance of excessive PN levels in the plasma also serves to avoid the generation of undesirably high PN level inside the tissue cells, where due to the action of kinase the conversion of PL into the desired intracellular PLP occurs. It is now realised for the first time that this crucial phosphorylation step inside the cells (where the intracellular PLP is vital to a number of biochemical reactions) is also inhibited by xanthine bronchodilators. This same kinase also controls the phosphorylation of PN to PNP. PN therefore competes with PL for the available kinase. An excessive intracellular supply of PN will therefore on the one hand suppress the formation of intracellular PLP even further and would thus strongly augment the already present enzyme inhibition by xanthine bronchodilators. On the other hand, high intracellular PNP levels arising from oversupply of free PN to the cells, will also compete with PLP for vital enzyme sites, thus further suppressing intracellular B6 activity. There exists no known alternative route by which intracellular PLP can be generated to compensate for the resultant deficiency.
m) In contrast to previously held views, it is now realised that a patient's ability to correct any deficiencies of plasma-PLP values due to previously known mechanisms involving certain theophylline derivatives, is not compromised for as long as the vitamin B6 metabolising enzymes have not been affected. However, the newly discovered inhibition of PL-kinase, caused even by theophylline itself (as opposed to its derivative aminophylline) will compromise the patient's ability to correct the intracellular deficit, both in the liver and in other cells.
n) In addition, the applicant's enzyme kinetic studies have shown that PL-kinase inhibition by xanthine bronchodilators is of the non-competitive type. This means that the long-term administration of xanthine bronchodilators alone will gradually lead to a depletion of enzyme reserves.

The efficacy with which red blood cells (RBC) can deliver oxygen to tissues is determined by the oxygen binding capacity of the RBC: the greater the oxygen binding capacity, the lower the rate of oxygen delivery to the tissues will be.

In asthma, tissue oxygen delivery is already compromised due to the obstructive air passages.

Theophylline and other xanthine bronchodilators while on the one hand improving the situation as a result of bronchodilation, also tend to aggravate tissue oxygen deprivation on the other hand due to its preferential effects on B6 vitamin metabolism as shown below. This unfavourable effect of xanthine bronchodilators can be effectively reversed by the concomitant administration of Vitamin B6 in the correct dosage form and formulation and dosage level as shown herein.

From the aforegoing it will be apparent that the present invention is based on more than a mere additive effect of known properties of xanthine bronchodilators and Vitamin B6. There is in fact substantial previously unknown mutual interaction, whereby B6 inter alia potentiates the desired therapeutic effects.

PLP is less preferred. Applicant has now established that - in contrast to presently held views in the art - the body produces special phosphatases (to be named "PLP-phosphatase") on demand for the hydrolysis of PLP (as a storage form) to PL. The applicant has identified two different PLP-phosphatases in RBC, one of which also occurs in plasma. Moreover, and quite unexpectedly, the applicant has also now found that these PLP-phosphatases are inhibited by disease-produced toxins which are present in the plasma of seriously ill patients (but not in plasma from normal healthy controls). Thus, in serious disease, such as seriously ill asthma patients, the patients' ability to hydrolyse PLP to PL becomes compromised. Under these circumstances, the administration of PL as such to the patient is particularly beneficial.

The present invention provides the means for a method of lowering serotonin and/or histamine levels and/or central nervous system side effects induced by xanthine bronchodilators which comprises managing intracellular and plasma levels of pyridoxal and phosphorylated pyridoxal. Preferably said managing is effected by supplementation with pyridoxal.

The following examples are to be read against the background of the preceding general disclosure of the invention and are intended to enable the skilled addressee to practise the invention as generally disclosed.

### Examples

In the following examples 1 to 3 pyridoxine hydrochloride is granulated with a pharmaceutical grade dicalcium phosphate filler (available in the trade under the trade mark EMCOMPRESS), serving as an inert insoluble filler. The theophylline is granulated separately with lactose, which is a soluble filler. Thereafter the two types of granules are mixed with one another and with a synthetic high molecular weight polymer of acrylic acid, cross-linked with allyl sucrose containing 56 to 68% carboxylic acid groups (this carboxy vinyl polymer, available in the trade under the trade mark CARBOPOL 934 forms a gel with dicalcium phosphate in the presence of water) and a commercial grade hydroxy ethyl cellulose (available in the trade under the trade mark NATROSOL) and pressed into tablets. Varying dissolution characteristics can be obtained by changing the proportions of the tabletting aids, various compositions being exemplified in the following:

### Example 1

Tablets were formed having the following composition (all quantities in mg/tablet): pyridoxine hydrochloride 10, dicalcium phosphate 125, theophylline 200, carboxyl vinyl polymer 100, hydroxy ethylcellulose 125, magnesium stearate 6 and 5% gelatin solution QS.

### Method:

The pyridoxine HCl and dicalcium phosphate are granulatd with the aid of the 5% gelatin. The theophylline and lactose are similarly and separately granulated with 5% gelatin. The two granulates are mixed with one another and with carboxy vinyl polymer, hydroxy ethylcellulose and magnesium stearate and tabletted at 70 N pressure.

The normal dosage for adults is 3 to 4 tablets per day. Half that dosage is administered to children.

The above tablets were tested for dissolution rates in water in the usual manner by recording adsorption at 324 nm for pyridoxine and at 285 nm for theophylline with the following results:

| Time (min) | Percentage of dose released | |
|---|---|---|
| | pyridoxine | theophylline |
| 0 | 0 | 0 |
| 100 | 40 | 15 |
| 200 | 65 | 30 |
| 300 | 80 | 50 |
| 400 | 90 | 60 |
| 480 | | 70 |
| The above release rates are considered ideal. | | |

### Example 2

Tablets having substantially the same dissolution characteristics as in Example 1 were prepared by the method in accordance with Example 1, having the following composition, all quantities in mg/tablet:
pyridoxine hydrochloride 20, dicalcium phosphate 125, theophylline 375, lactose 190, carboxyvinyl polymer 100, hydroxyethyl cellulose 125, magnesium stearate 6, 5% gelatin QS.

The normal dosage for adults is 2 tablets.

### Example 3

Tablets were prepared as in Example 1, but this time also containing zinc, in the form of zinc chloride and riboflavin, having the following composition (mg/tablet): pyridoxine hydrochloride 10, dicalcium phosphate 125, theophylline 200, lactose 100, carboxyvinyl polymer 100, hydroxy ethylcellulose 125, magnesium stearate 6, zinc chloride 10,0 (=4,8 mg Zn) riboflavin 1,0.

The method according to Example 1 was modified by the incorporation of the zinc chloride and the riboflavin in the pyridoxine granulate. The dissolution characteristics were substantially as in Example 1.

In all of the above examples the tablets are preferably pressed into notched circular tablets, having a single central score line or elongate tabules having one or two score lines whereby the tabule can be broken up into halves or thirds which are particularly useful for dose adjustment in children. For adults, similar tablets or elongate tablets with the same characteristics may be preferred of which the following is an example:

### Example 4

| | |
|---|---|
| Pyridoxine HCl | 20 mg |
| Dicalcium phosphate | 125 mg |
| Theophylline | 400 mg |
| Lactose | 150 mg |
| Carboxy vinyl polymer | 100 mg |
| Hydroxy ethylcellulose | 150 mg |
| Magnesium stearate | 6 mg |
| Zinc chloride | 20,0 mg |
| Riboflavin | 2,0 mg |

The method and dissolution characteristics are as in Example 3.

The recommended dosages are as in Example 1.

### Example 5

Composition (mg/tab): pyridoxine 10,0, dicalcium phosphate 250, gelatin (5%) QS, theophylline 200, hydroxy ethylcellulose 120, magnesium stearate 6.

### Method

The pyridoxine is mixed with the dicalcium phosphate, granulated with the gelatin solution and dried in an oven for approximately 2 hours.

The theophylline is mixed with hydroxy ethylcellulose and then mixed with the pyridoxine granules and the magnesium stearate and tabletted.

It will be noted that in this case the theophylline was not granulated separately and that the soluble filler (lactose) was omitted. These tablets have different dissolution characteristics in that between 30 and 40% of the active ingredients are dissolved within a few minutes followed by an almost linear dissolution rate until about 80 to 90% have been dissolved after 8 hours.

Alternatively a certain percentage (e.g. 30%) may be incorporated as free theophylline (as in example 5) while the rest of the theophylline dose is incorporated as a lactose-granulate (as in example 4) thus to provide a tablet with yet other dissolution characteristics. Other variations based on these principles are obvious.

Although the aforegoing examples are confined to slow release oral galenic forms it will be understood that (in a manner known per se) similar effects may be attained if the daily dosages herein taught are administered by infusion or in various suitable sustained-release or depot forms of the drug combination.

The recommended dosage rate is as in Example 1.

### Example 6

Examples 1 to 6 are repeated but replacing the pyridoxine hydrochloride in each case by an equivalent amount of pyridoxal hydrochloride. Within limits of experimental error the slow-release properties are substantially the same as when pyridoxine is used. The recommended dosages are also the same as in examples 1 to 6. Riboflavine can be omitted in all the formulations, when employing pyridoxal instead of pyridoxine. Instead an antioxidant such as vitamin C is preferably included.

### Example 7

Examples 1 to 6 are repeated, using pyridoxamine (PM) instead of pyridoxine, again the recommended dosages are the same as in examples 1 to 6.

### Example 8

All the aforegoing examples may be repeated, using enprofylline instead of theophylline or aminophylline. The content of enprophylline can in this case be reduced to 1/4 the theophylline levels in the earlier examples. Corresponding adjustments are made to the amount and composition of soluble filler (where applicable) used to regulate the release rate.

### Example 9

| Formulation (mg/tablet) | |
|---|---|
| Theophylline (anhydrous) BP | 375 |
| Pyridoxal HCl | 18.3 |
| Encompress | 50 |
| Eudragit RSPM (Rohm Pharma GMBH) | 15 |
| Ethocel 10 cps (Alchemist) | 5 |
| Lactose anhydrous | 50 |
| Talc BP | 5 |
| Magnesium stearate BP 1% w/w | 5 |
| Sodium carboxymethyl cellulose 2% w/v in water | - |
| Total mass | 5̅2̅3̅.̅3̅ |

### Manufacturing methodology. Direct compression/granulation

1. Mix theophylline and lactose (anhydrous) and add sufficient amount of 2% binder (2% w/v sod-carboxymethyl cellulose) in a mixer in order to get the wet mass for granulation. Pass the wet mass through a 2 mm mesh sieve and dry at 45°C for 5 hours. Pass the dried granules through a 710 »m sieve.
2. Mix a portion of fine granules with pyridoxal HCl in equal portions and mix with the rest of granules in either a cube mixer or revolving mixer (15 minutes at 20 rpm).
3. Mix Encompress, Eudragit RS, ethocel and talc and add to number two and mix for 15 minutes at 20 rpm.
4. Add magnesium stearate by sieving it directly through a 90 mesh screen on to the number 3 and mix for 8 minutes at 20 rpm.
5. Carefully fit the punches and die provided and set to the required mass and proceed with compression.

### Precautionary notes

1. Avoid contact with moisture after addition of pyridoxal HCl to the mass of granules.
2. The moisture content of the granules should be <4% w/w.
3. Protect against light.
4. Tablets should be compressed at high compression force in order to have the required hardness.

### Example 10

(A) Methods
Studies on humans: The study was approved by the University Human Ethics Committee. Seven apparently healthy, young male volunteers aged 23 ± 2.4 ( ± SD) years gave informed consent to participate in the study. Each participant received theophylline (slow release) at a dosage of approximately 5 mg/kg body weight/day for one week. Thereafter, the theophylline dosage was increased to maintain plasma theophylline levels of approx. 10 mg/liter for 15 weeks. During the last week of the study (16th week) the participants were supplemented with 10 mg of vitamin B6 (B6) as herein described per day. Venous blood samples with EDTA as anticoagent were obtained 12 times from the participants, viz. day 1, 5, 12, 22, 32, 42, 52, 66, 80, 94, 105 and 112.
Serotonin was extracted from urine using the extraction method of Maruyama, Y. and A.E. Takemori (1971) Biochem. Pharmacol. 20: 1833-1841 as modified by Tsuchiya, H, T. Hayashi, M. Tatsumi, Y. Hoshino, S. Ohtani and N. Takagi. 1989. Clin. Chem. 35: 43-47. Serotonin concentrations were subsequently determined by HPLC according to the method of Tsuchiya et al.
(B) Results
Theophylline therapy resulted in a significant increase in basal urinary serotonin excretion after 6 and 15 weeks of theophylline therapy (see table). One week of B6 supplementation returned urinary serotonin excretion to pre-treatment levels. Urinary serotonin excretion after tryptophan loading followed the same trend as basal serotonin excretion, but the observed changes were not statistically significant.
(C) Table
Effect of the theophylline therapy on urinary serotonin.

| Days of theophylline treatment | Urinary serotonin excretion (»mol/24 h) Basal |
|---|---|
| 0 | 0.503 (0.198) |
| 42 | *0.707 (0.203) |
| 105 | *0.730 (0.146) |
| 112 | 0.562 (0.127) |

| | |
|---|---|
| *Compared to initial levels, basal urinary serotonin excretion increased significantly (p = 0.016) during theophylline treatment. | |

Measurements were preceded by 1 week of vitamin B6 supplementation (10 mg/day).

## Claims

1. The use of vitamin B6 for the production of a pharmaceutical composition which is intended to counteract elevated serotonin levels in plasma and urine and serotonin associated side effects arising in the treatment or prophylaxis of obstructive air passage disease with a xanthine bronchodilator, such as
a) decreased responsiveness to the xanthine bronchodilator;
b) CNS side effects.

2. The use as claimed in claim 1, characterised in that the vitamin B6, preferably in a slow or sustained release form, is in the form of pyridoxal, preferably in combination with an antioxidant, or in the form of a compound which in vivo, once it has entered the bloodstream, is rapidly converted into pyridoxal without the intervention of oxidase or oxygen, preferably pyridoxal itself or a complex or acid addition salt thereof, or is in the form of pyridoxine or a complex or acid addition salt of pyridoxine, provided that such pyridoxine, or complex or addition salt thereof is always galenically presented in a slow or sustained release form.

3. The use as claimed in claim 1 or 2, characterised in that the vitamin B6 is provided in a combination with said xanthine bronchodilator, as dosage units, but as separate and distinct compounds, and that the dose by weight of vitamin B6 is substantially lower than that of the xanthine bronchodilator, preferably in a ratio of vitamin B6 to xanthine bronchodilator of 1:2 to 1:70, more preferably 1:8 to 1:30, and wherein preferably the vitamin B6 as well as the xanthine bronchodilator are incorporated in a slow or sustained release form.

4. The use as claimed in any one or more of claims 1 to 3, characterised in that the xanthine bronchodilator is theophylline or enprophylline.

5. The use as claimed in any one or more of claims 1 to 4, characterized in that the pharmaceutical composition produced also serves to prevent or counteract xanthine bronchodilator-induced lowering of glutamate pyruvate transaminase in red blood cells.

6. The use as claimed in any one or more of claims 1 to 5, characterized in that the pharmaceutical composition produced also serves to prevent or counteract elevated histamine levels induced by the xanthine bronchodilator.

7. The use as claimed in any one or more of claims 1 to 6, characterized in that the pharmaceutical composition produced also serves to prevent or counteract elevated homocysteine levels and effects thereof, induced by the xanthine bronchodilator.

## Patentansprüche

1. Verwendung von Vitamin B6 für die Herstellung einer pharmazeutischen Zusammensetzung, die dem Zweck dient, überhöhten Serotoninspiegeln in Plasma und Urin, sowie mit Serotonin zusammenhängenden Nebenwirkungen, zu begegnen, die sich aus der Behandlung oder Prophylaxe von obstructiven Atemwegserkrankungen mit einem Xanthin-Bronchodilator ergeben, wie
a) verringerte Ansprechbarkeit auf den Xanthin-Bronchodilator;
b) ZNS-Nebenwirkungen.

2. Verwendung gemäß Anspruch 1, dadurch gekennzeichnet, daß das Vitamin B6, vorzugsweise in einer Retardformulierung, als Pyridoxal vorliegt, vorzugsweise in Kombination mit einem Anti-oxydanten, oder als eine Verbindung, die in vivo, nach Eintritt in den Blutkreislauf schnell in Pyridoxal umgewandelt wird ohne die Beteiligung von Oxydase oder Sauerstoff, vorzugsweise Pyridoxal selbst oder ein Komplex oder ein Säureadditionssalz desselben, oder in Form von Pyridoxin oder einem Komplex oder einem Säureadditionssalz von Pyridoxin vorliegt, mit der Maßgabe, daß solches Pyridoxin, oder dessen Komplex oder Additionssalz, stets galenisch als Retardform formuliert ist.

3. Verwendung gemäß Anspruch 1 oder 2, dadurch gekennzeichnet, daß das Vitamin B6 und der Xanthin-Bronchodilator in Kombination miteinander als Dosiereinheiten, jedoch als getrennt vorliegende und unterschiedliche Verbindungen angewandt werden, und daß die gewichtsbezogene Dosierung für Vitamin B6 erheblich geringer ist als die des Xanthin-Bronchodilators, vorzugsweise in einem Verhältnis von Vitamin B6 zum Xanthin-Bronchodilator von 1:2 bis 1:70, mehr bevorzugt 1:8 bis 1:30, und worin vorzugsweise sowohl das Vitamin B6 als auch der Xanthin-Bronchodilator als Retardformulierung vorliegen.

4. Verwendung gemäß einem oder mehreren der Ansprüche 1 bis 3, dadurch gekennzeichnet, daß der Xanthin-Bronchodilator Theophyllin oder Enprophyllin ist.

5. Verwendung gemäß einem oder mehreren der Ansprüche 1 bis 4, dadurch gekennzeichnet, daß das hergestellte pharmazeutische Präparat auch dazu dient, eine Xanthin-Bronchodilator-induzierte Senkung von Glutamatpyruvattransaminase in roten Blutzellen zu verhindern oder ihr entgegenzuwirken.

6. Verwendung gemäß einem oder mehreren der Ansprüche 1 bis 5, dadurch gekennzeichnet, daß das so hergestellte pharmazeutische Präparat auch dazu dient, vom Xanthin-Bronchodilator induzierte erhöhte Histaminspiegel zu verhindern oder diesen entgegenzuwirken.

7. Verwendung gemäß einem oder mehreren der Ansprüche 1 bis 6, dadurch gekennzeichnet, daß das so hergestellte pharmazeutische Präparat auch dazu dient, erhöhte Homocysteinspiegel und deren Auswirkungen, die vom Xanthin-Bronchodilator induziert wurden, zu verhindern oder diesen entgegenzuwirken.

## Revendications

1. Utilisation de vitamine B6 pour la production d'une composition pharmaceutique qui est prévue pour neutraliser des niveaux élevés de sérotonine dans le plasma et l'urine et des effets secondaires associés à la sérotonine apparaissant lors du traitement ou de la prophylaxie des maladies respiratoires obstructives avec un bronchodilatateur à la xanthine, tel que
a) une réaction diminuée au bronchodilatateur à la xanthine ;
b) Effets secondaires CNS.

2. Utilisation selon la revendication 1, caractérisée en ce que la vitamine B6, de préférence sous forme de libération lente ou prolongée, est sous la forme de pyridoxal, de préférence en combinaison avec un antioxydant, ou sous la forme d'un composé qui, in vivo, une fois qu'il est entré dans la circulation sanguine, est rapidement converti en pyridoxal sans l'intervention d'oxydase ou d'oxygène, de préférence du pyridoxal lui-même ou un complexe ou un sel d'addition acide de celui-ci, ou est sous la forme de pyridoxine ou d'un complexe ou d'un sel d'addition acide de pyridoxine, à condition qu'une telle pyridoxine, ou qu'un tel complexe ou sel d'addition de celle-ci se présente toujours galéniquement sous forme de libération lente ou prolongée.

3. Utilisation selon la revendication 1 ou 2, caractérisée en ce que la vitamine B6 est fournie dans une combinaison avec ledit bronchodilatateur à la xanthine, comme unités de dosage, mais comme composés séparés et distincts, et en ce que le dosage en poids de vitamine B6 est substantiellement inférieur à celui du bronchodilatateur à la xanthine, de préférence dans un rapport de vitamine B6 au bronchodilatateur à la xanthine de 1:2 à 1:70, plus préféré 1:8 à 1:30, et dans laquelle de préférence la vitamine B6 de même que le bronchodilatateur à la xanthine sont incorporés sous forme de libération lente ou prolongée.

4. Utilisation selon l'une quelconque ou plus des revendications 1 à 3, caractérisée en ce que le bronchodilatateur à la xanthine est de la théophylline ou de l'enprophylline.

5. Utilisation selon l'une quelconque ou plus des revendications 1 à 4, caractérisée en ce que la composition pharmaceutique produite sert aussi à empêcher ou à neutraliser la baisse de transaminase de pyruvate glutamate dans les globules rouges induite par le bronchodilatateur à la xanthine.

6. Utilisation selon l'une quelconque ou plus des revendications 1 à 5, caractérisée en ce que la composition pharmaceutique produite sert aussi à empêcher ou à neutraliser des niveaux d'histamine élevés induits par le bronchodilatateur à la xanthine.

7. Utilisation selon l'une quelconque ou plus des revendications 1 à 6, caractérisée en ce que la composition produite sert également à empêcher ou à neutraliser des niveaux d'homocystéine élevés et des effets de ceux-ci, induits par le bronchodilatateur à la xanthine.
